# EUROPEAN PATENT SPECIFICATION

(11) **EP 4 374 890 B1**
(45) Date of publication and mention of the grant of the patent: **28.01.2026**
(21) Application number: 22212481.0
(22) Date of filing: 09.12.2022
(51) Int. Cl.: A61M 5/142, A61M 5/158, A61M 25/06

(54) **MEDICAL INSERTION MECHANISM AND MEDICAL DEVICE THEREWITH**
MEDIZINISCHER EINSETZMECHANISMUS UND DAZUGEHÖRIGE MEDIZINISCHE VORRICHTUNG
MÉCANISME D'INSERTION MÉDICAL ET DISPOSITIF MÉDICAL LE COMPRENANT

(30) Priority: 23.11.2022 US 202217992954
(43) Date of publication of application: 29.05.2024
(73) Proprietor: Altek Biotechnology Corporation, 300 Hsinchu City (TW)
(72) Inventor: Huang, Yu-Cheng, 300 Hsinchu City (TW)
(74) Representative: Straus, Alexander

(56) References cited:
- US-A1- 2005 245 956
- US-A1- 2015 196 719
- US-A1- 2021 220 555

## Description

### Field of the Invention

The present invention relates to a medical insertion mechanism and a medical device therewith according to the pre-characterizing clauses of claims 1 and 13.

### Background of the Invention

A medical device, e.g., an on-body injector, is designed to deliver a dose of a drug. However, the conventional on-body injectors available in the markets fail to meet requirements of simple structure, compact size, easy operation and precise insertion and retraction function. Therefore, an improvement of the medical device is urgently needed. US 2021/220555 discloses such an on-body injector with an insertion and retraction function according to the state of the art.

### Summary of the Invention

This in mind, the present invention aims at providing a medical insertion mechanism with simple structure, compact size, easy operation and precise insertion and retraction function, and a medical device therewith.

This is achieved by a medical insertion mechanism and a medical device therewith according to claims 1 and 13. The dependent claims pertain to corresponding further developments and improvements.

As will be seen more clearly from the detailed description following below, the claimed medical insertion mechanism includes a base, a needle holder, a needle, a cannula holder, a cannula, a pivoting assembly, a lever, an triggering component, a driving component and a column component. The needle holder is disposed on the base and slidable relative to the base along an insertion direction or a retraction direction opposite to the insertion direction. The needle holder includes a pushing structure. The needle is disposed on the needle holder. The cannula holder is arranged at a position corresponding to the needle holder. The pushing structure is configured to abut against the cannula holder along the insertion direction. The cannula is disposed on the cannula holder. The pivoting assembly is disposed on the base and pivotable relative to the base from a first position through a second position to a third position. The lever is movably disposed on the base and movably engaged with the pivoting assembly and the needle holder. The triggering component is slidably disposed on the base. The triggering component includes an abutting structure configured to slidably engage with or disengage from the pivoting assembly. The driving component is configured to drive the pivoting assembly to pivot from the first position through the second position to the third position when the abutting structure slidably disengages from the pivoting assembly. When the pivoting assembly pivots from the first position to the second position, the lever drives the needle holder and the cannula holder to slide along the insertion direction, so as to drive the needle and the cannula to move along the insertion direction together. When the pivoting assembly pivots from the second position to the third position, the lever drives the needle holder to slide along the retraction direction to drive the needle to retract relative to the cannula. The column component is detachably installed on the base and for resiliently deforming the driving component. The driving component is a spring at least partially sleeved on the column component, and two ends of the driving component respectively abut against the pivoting assembly and the column component. The column component, the driving component, the cannula holder and the needle holder are located at a first side of the lever, and the abutting structure is located at a second side of the lever.

Furthermore, the claimed medical device includes a case and the aforementioned medical insertion mechanism, and the base of the medical insertion mechanism is disposed on the case.

In summary, the present invention has less parts and compact size. Besides, in the present invention, when it is desired to use the medical device for drug injection or fluid drainage, it only has to operate the operating component to drive the triggering component to slide for slidably disengaging the abutting structure of the triggering component from the protruding structure of the pivoting assembly, so that the cannula can be inserted into the patient's body. Therefore, the present invention has easy operation and precise insertion and retraction function.

These and other objectives of the present invention will no doubt become obvious to those of ordinary skill in the art after reading the following detailed description of the preferred embodiment that is illustrated in the various figures and drawings.

### Brief Description of the Drawings

In the following, the invention is further illustrated by way of example, taking reference to the accompanying drawings. Thereof
FIG. 1 is a block diagram of a medical device according to an embodiment of the present invention,
FIG. 2 and FIG. 3 are partial diagrams of the medical device at different views according to the embodiment of the present invention,
FIG. 4 and FIG. 5 are partial exploded diagrams of the medical device at different views according to the embodiment of the present invention,
FIG. 6 is a partial sectional diagram of the medical device according to the embodiment of the present invention,
FIG. 7 is another partial diagram of the medical device according to the embodiment of the present invention,
FIG. 8 is another partial sectional diagram of the medical device according to the embodiment of the present invention,
FIG. 9 is a partial diagram of the medical device in a first state according to the embodiment of the present invention,
FIG. 10 is a partial diagram of the medical device in a second state according to the embodiment of the present invention,
FIG. 11 is a partial diagram of the medical device in a third state according to the embodiment of the present invention,
FIG. 12 is a partial diagram of the medical device in a fourth state according to the embodiment of the present invention, and
FIG. 13 and FIG. 14 are partial diagrams of a medical device in different states according to another embodiment of the present invention.

### Detailed Description

In the following detailed description of the preferred embodiments, reference is made to the accompanying drawings which form a part hereof, and in which is shown by way of illustration specific embodiments in which the invention may be practiced. In this regard, directional terminology, such as "top", "bottom", "left", "right", "front", "back", etc., is used with reference to the orientation of the Figure(s) being described. The components of the present invention can be positioned in a number of different orientations. As such, the directional terminology is used for purposes of illustration and is in no way limiting. Accordingly, the drawings and descriptions will be regarded as illustrative in nature and not as restrictive. Also, if not specified, the term "connect" or "couple" is intended to mean either an indirect or direct electrical/mechanical connection. Thus, if a first device is connected or coupled to a second device, that connection may be through a direct electrical/mechanical connection, or through an indirect electrical/mechanical connection via other devices and connections.

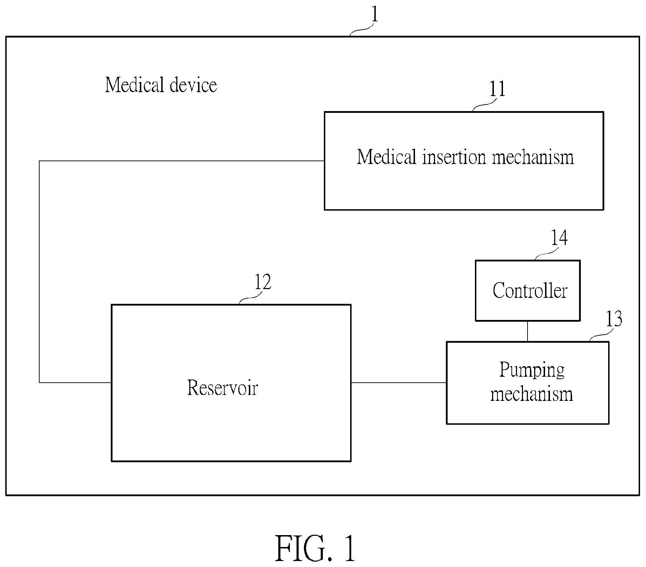

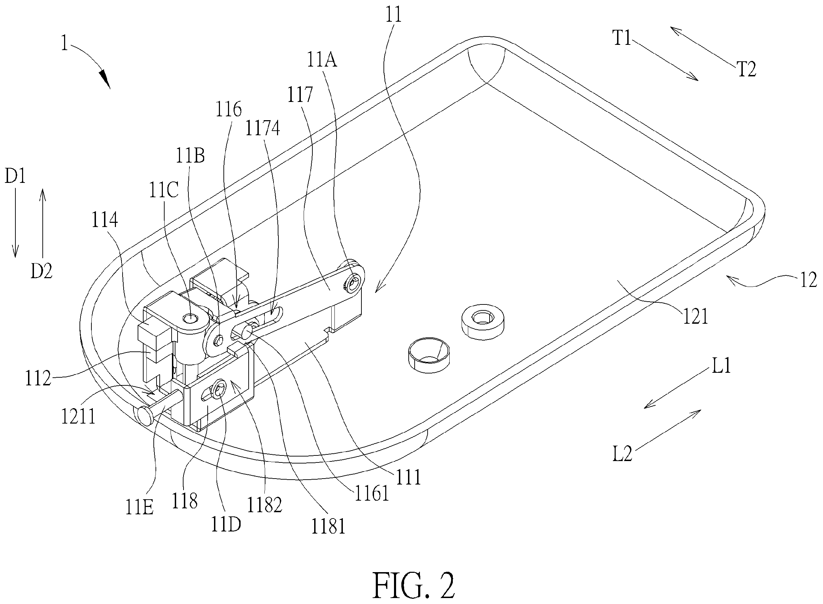

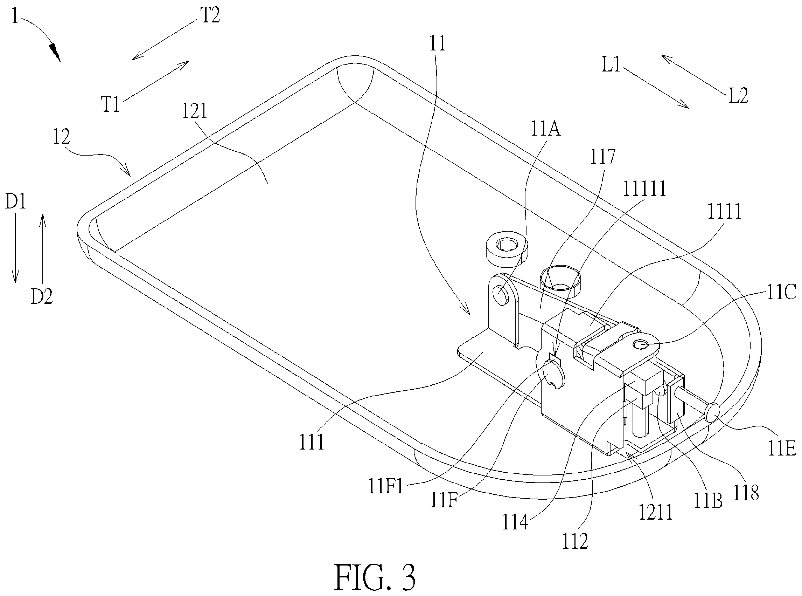

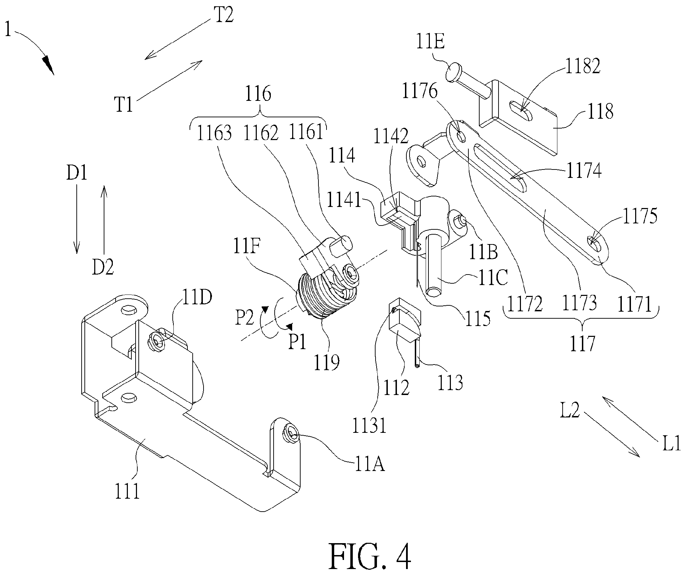

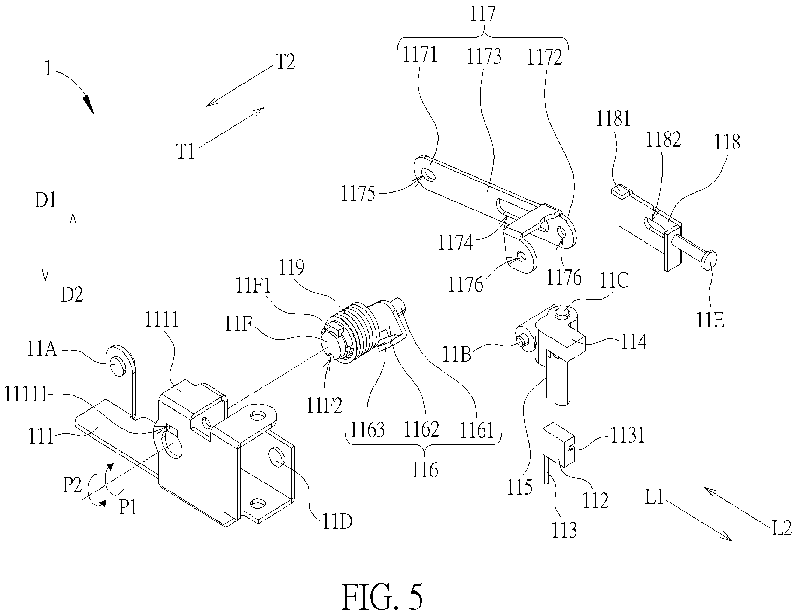

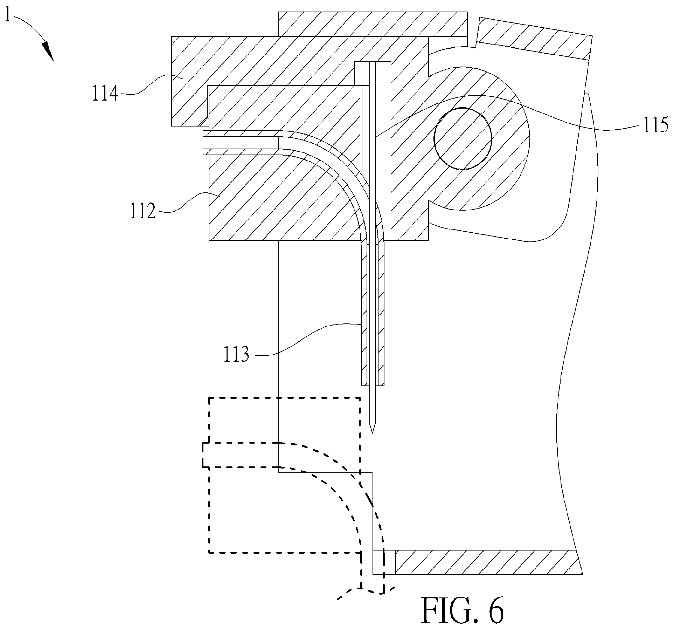

Please refer to FIG. 1 to FIG. 6. FIG. 1 is a block diagram of a medical device 1 according to an embodiment of the present invention. FIG. 2 and FIG. 3 are partial diagrams of the medical device 1 at different views according to the embodiment of the present invention. FIG. 4 and FIG. 5 are partial exploded diagrams of the medical device 1 at different views according to the embodiment of the present invention. FIG. 6 is a partial sectional diagram of the medical device 1 according to the embodiment of the present invention. As shown in FIG. 1 to FIG. 6, in this embodiment, the medical device 1 can be an on-body injector for drug injection. However, the present invention is not limited to this embodiment. For example, in another embodiment, the medical device can be a trocar for fluid drainage. The medical device 1 includes a medical insertion mechanism 11. The medical insertion mechanism 11 includes a base 111, a cannula holder 112, a cannula 113, a needle holder 114, a needle 115, a pivoting assembly 116, a lever 117, an triggering component 118 and a driving component 119. The needle holder 114 is disposed on the base 111 and slidable relative to the base 111 along an insertion direction D1 or a retraction direction D2 opposite to the insertion direction D1. The needle holder 114 includes a pushing structure 1141. The needle 115 is disposed on the needle holder 114. The cannula holder 112 is arranged at a position corresponding to the needle holder 114. The pushing structure 1141 is configured to abut against the cannula holder 112 along the insertion direction D1. The cannula 113 is disposed on the cannula holder 112. The cannula 113 is configured to provide a drug passage or a fluid passage. In this embodiment, as shown in FIG. 4, a positioning recess 1142 can be formed on the needle holder 114 for at least partially receiving the cannula holder 112, and the pushing structure 1141 can be a wall of the positioning recess 1142. Besides, as shown in FIG. 6, in this embodiment, the needle 15 can be a hollow structure or a solid structure at least partially disposed inside the cannula 113 in a removable penetrating manner, and the cannula 113 can be made of a resealable material for preventing leakage. However, the present invention is not limited to this embodiment.

In this embodiment, in order for drug injection, as shown in FIG. 1, the medical device 1 further includes a reservoir 12, a pumping mechanism 13 and a controller 14. The reservoir 12 is for drug storage and is communicated with the cannula 113 via a flexible tube connected between a port 1131 of the cannula 113 and a port of the reservoir 12. The controller 12 is configured to control the pumping mechanism 13 to pump a drug to flow from the reservoir 13 to the cannula 113 through the flexible tube. The pumping mechanism 13 can but not limited to include an electric motor, a plunger slidably disposed in the reservoir 12 and a reducer connected between the electric motor and the plunger. The controller 14 can be a micro processing unit (MCU), a processor, or an application specific integrated circuit (ASIC) for controlling the motor to drive the plunger to slide to pump the drug out of the reservoir 12. However, the structures of the pumping mechanism and the medical device are not limited to this embodiment. It depends on practical demands.

Besides, as shown in FIG. 2 to FIG. 5, the pivoting assembly 116 is disposed on the base 111 and pivotable relative to the base 111. The lever 117 is movably disposed on the base 111 and movably engaged with the pivoting assembly 116 and the needle holder 114. The triggering component 118 is disposed on the base 111 and slidable relative to the base 111 along an unlocking direction L1 perpendicular to the insertion direction D1, or along a locking direction L2 opposite to the unlocking direction L1. The triggering component 118 includes an abutting structure 1181 configured to slidably engage with the pivoting assembly 116 along the locking direction L2 or disengage from the pivoting assembly 116 along the unlocking direction L1. The driving component 119 is configured to drive the pivoting assembly 116 to pivot along a first pivoting direction P1 around a pivoting axis perpendicular to the unlocking direction L1 and the insertion direction D1 when the abutting structure 1181 slidably disengages from the pivoting assembly 116. When the pivoting assembly 116 pivots along the first pivoting direction P1, the lever 117 drives the needle holder 114 to slide along the insertion direction D1 and then to slide along the retraction direction D2. When the lever 117 drives the needle holder 114 to slide along the insertion direction D1, the needle holder 114 pushes the cannula holder 112 to slide together with the needle holder 114 by the pushing structure 1141, so as to drive the needle 115 and the cannula 113 to move along the insertion direction D1 together for insertion of the needle 115 and the cannula 113 into a patient's body along the insertion direction D1. When the lever 117 drives the needle holder 114 to slide along the retraction direction D2, the lever 117 drives the needle holder 114 to slide along the retraction direction D2 to drive the needle 115 to retract relative to the cannula 113 along the retraction direction D2 for withdrawing the needle 115 from the patient's body along the retraction direction D2 and leaving the cannula 113 inserted in the patient's body.

In order to improve convenience of use, the cannula 113 can be made of flexible material. The needle 115 can be made of rigid material, and a length of the cannula 113 along a direction parallel to the insertion direction D1 and the retraction direction D2 can be less than a length of the needle 115 along the direction parallel to the insertion direction D1 and the retraction direction D2.

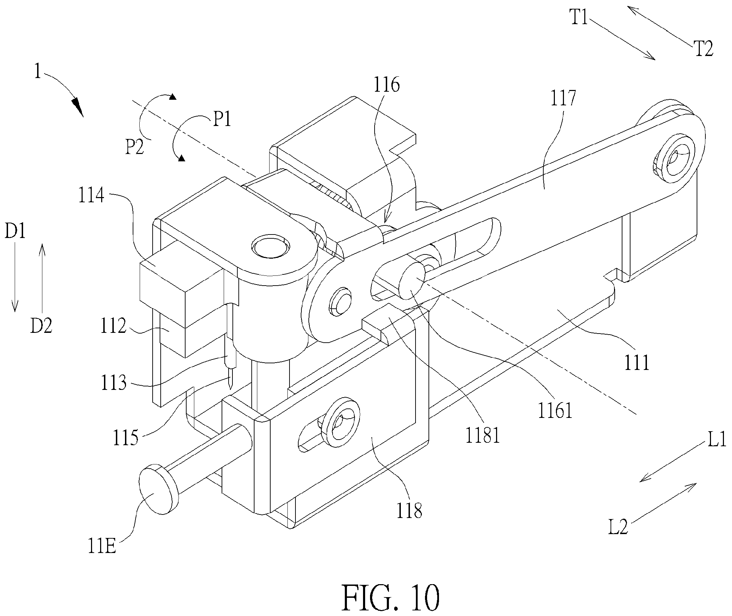

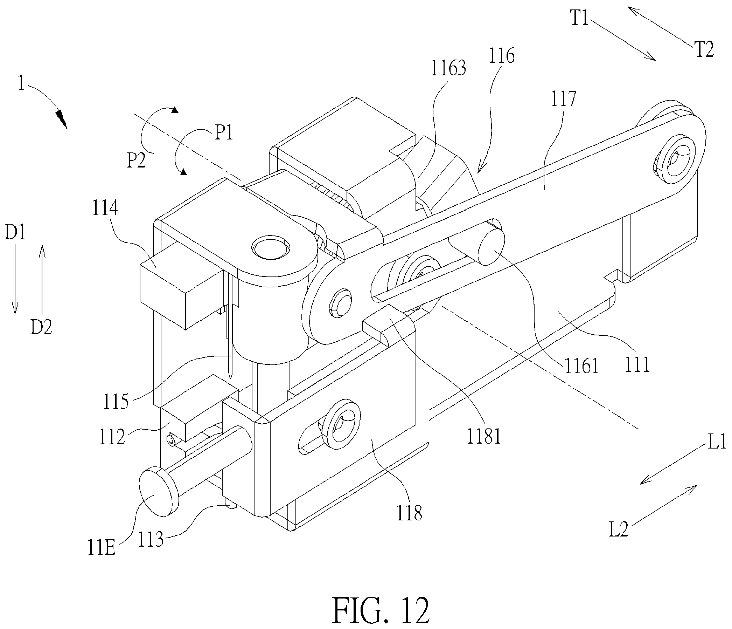

Furthermore, in this embodiment, the lever 117 can be driven by the pivoting assembly 116 to drive the needle holder 114 to slide from an original position (as shown in FIG. 10) along the insertion direction D1 and then back to the original position (as shown in FIG. 12) along the retraction direction D2 when the pivoting assembly 116 pivots along the first pivoting direction P1. However, the present invention is not limited to this embodiment. For example, in another embodiment, the lever can be configured to drive the needle holder to slide from the original position and then back to a non-original position which may be higher or lower than the original position when the pivoting assembly pivots along the first pivoting direction.

As shown in FIG. 2 and FIG. 3, the medical device 1 further includes a case 12 for preventing damage of the medical insertion mechanism 11. The case 12 includes a first mounting part 121 and a second mounting part, which is not shown in the figures, detachable installed on the first mounting part 121. The first mounting part 121 has an aperture 1211, so that the needle 115 and the cannula 113 can pass through the aperture 1121 to be inserted into the patient's body. The base 111 is mounted on the first mounting part 121. However, the present invention is not limited to this embodiment.

As shown in FIG. 2 to FIG. 5, the lever 117 is located between the pivoting assembly 116 and the triggering component 118 and includes a first end 1171, a second end 1172 and a middle portion 1173 between the first end 1171 and the second end 1172. The pivoting assembly 116 is engaged with a longitudinal slot 1174 formed on the middle portion 1173 of the lever 117 between a first end 1171 and a second end 1172 of the lever 117. The base 111 is movably connected to the first end 1171 of the lever 117. The needle holder 114 is movably connected to the second end 1172 of the lever 117.

Specifically, the longitudinal slot 1174 is adjacent to the second end 1172 of the lever 117 and away from the first end 1171 of the lever 117. The lever 117 has a first side and a second side opposite to the first side. The cannula holder 112 and the needle holder 114 are located at the first side of the lever 117. The pivoting assembly 116 includes a protruding structure 1161 passing through the longitudinal slot 1174 and movable relative to the longitudinal slot 1174, and the pivoting assembly 116 further includes a pivoting body 1162 located at the first side of the lever 117. The protruding structure 1161 protrudes from the pivoting body 1162. The abutting structure 1181 is located at the second side of the lever 117 and configured to abut against the protruding structure 1161. The aforementioned configuration can achieve compact size and labor saving. However, the present invention is not limited to this embodiment. For example, the longitudinal slot can be adjacent to the first end of the lever and away from the second end of the lever, and the needle holder and the cannula holder can be located at the second side of the lever.

Besides, an elongated hole 1175 is formed on the first end 1171 of the lever 117. Two circular holes 1176 are formed on the second end 1172 of the lever 117. The medical insertion mechanism 11 further includes a connecting pin 11A disposed on the base 111 and a driving pin 11B disposed on the needle holder 114. The connecting pin 11A passes through the elongated hole 1175 and is movable relative to the elongated hole 1175. Two ends of the driving pins 11B respectively pass through the circular holes 1176 and are movable relative to the circular holes 1176. By a cooperation of the connecting pin 11A and the elongated hole 1175, a cooperation of the driving pin 11B and the circular holes 1176, and a cooperation of the protruding structure 1161 and the longitudinal slot 1174, a pivoting movement of the pivoting assembly 116 relative to the base 111 can drive the lever 117 to pivot relative to the base 111 to drive the needle holder 114 to slide along the insertion direction D1 and/or the retraction direction D2. However, the present invention is not limited to this embodiment. For example, in another embodiment, there can be only one circular hole formed on the second end of the lever and one driving pin disposed on the needle holder. Alternatively, in another embodiment, the circular hole and the elongated hole can be respectively formed on the first end and the second end of the lever, and the connecting pin and the driving pin can respectively pass through the circular hole and the elongated hole and respectively be movable relative to the circular hole and the elongated hole. Alternatively, in another embodiment, the circular hole and the elongated hole can be respectively formed on the needle holder and the base, and the driving pin and the connecting pin can be respectively disposed on the second end of the lever and the first end of the lever.

As shown in FIG. 2 to FIG. 5, in other to ensure the needle 115 and the cannula 113 to be driven to move along the insertion direction D1 when the pivoting assembly 116 pivots along the first pivoting direction P1, the medical insertion mechanism 11 further includes a guiding component 11C disposed on the base 111 and configured to guide the needle holder 114 and the cannula holder 112 to slide along the insertion direction D1. In this embodiment, the guiding component 11C can be a guiding column passing through the needle holder 114. However, the present invention is not limited to this embodiment. For example, in another embodiment, the guiding component can be a guiding column passing through the needle holder and the cannula holder or passing through the cannula holder only, or the guiding component can be a guiding track configured to cooperate with at least one of the needle holder and the cannula holder.

Furthermore, in order to achieve compact size, in this embodiment, the lever 117 can be engaged with a portion of the needle holder 114 located between the pivoting assembly 116 and the guiding component 11C by the driving pin 11B. However, the present invention is not limited to this embodiment. For example, in another embodiment, the lever can be engaged with a portion of the needle holder located at a side of the guiding component away from the pivoting assembly.

As shown in FIG. 4 and FIG. 5, the pivoting assembly 116 further includes a stopping structure 1163 extending from the pivoting body 1162 and configured to contact with the base 111 for stopping the pivoting assembly 116 to prevent an excessive pivoting movement of the pivoting assembly 116, when the pivoting assembly 116 pivots along the first pivoting direction P1.

In addition, as shown in FIG. 2 to FIG. 5, in order to achieve a sliding configuration of the triggering component 118 relative to the base 111, a sliding slot 1182 is formed on the triggering component 118, the medical insertion mechanism 11 further includes a sliding pin 11D disposed on the base 111, and the sliding pin 11D passes through the sliding slot 1182 and is movable relative to the sliding slot 1182.

In this embodiment, the medical insertion mechanism 11 can further include an operating component 11E connected to the triggering component 118 and exposed out of the base 111 and/or the case 12 for easy manual operation. However, the present invention is not limited to this embodiment. For example, the triggering component can be driven to slide by a linking mechanism actuated by an electric motor or a pneumatic motor.

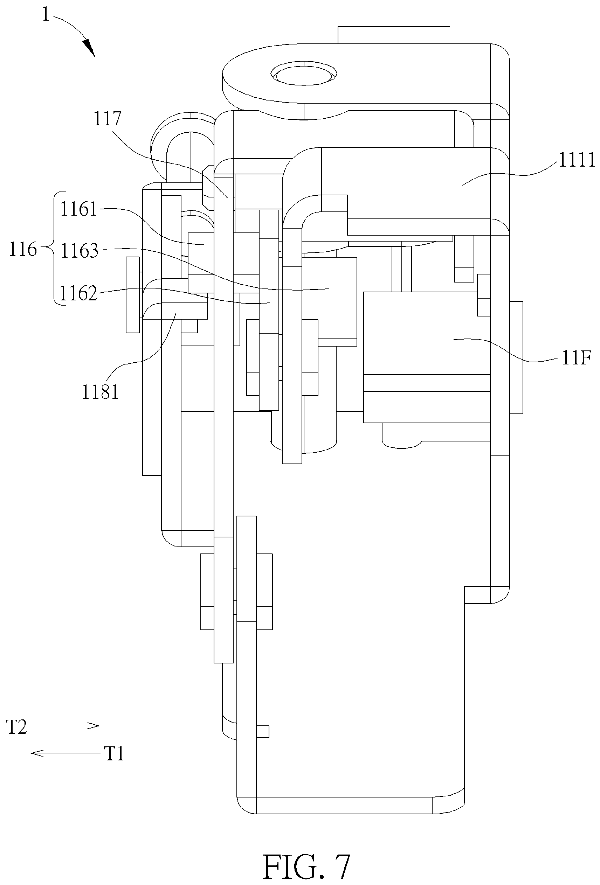

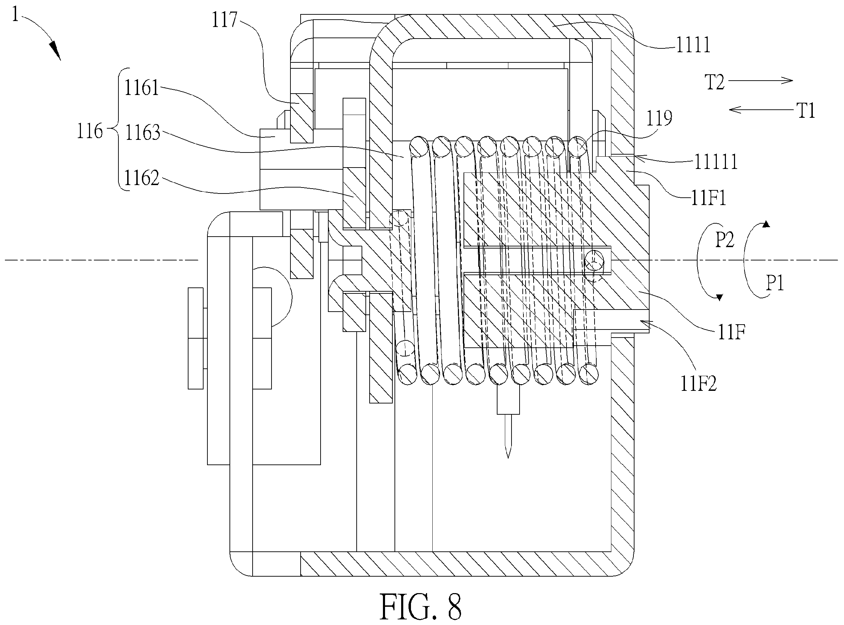

Please refer to FIG. 3 to FIG. 8. FIG. 7 is another partial diagram of the medical device 1 according to the embodiment of the present invention. FIG. 8 is another partial sectional diagram of the medical device 1 according to the embodiment of the present invention. As shown in FIG. 3 to FIG. 8, the medical insertion mechanism 11 further includes a column component 11F detachably installed on the base 111 and for resiliently deforming the driving component 119. In this embodiment, the driving component 119 can be a spring at least partially sleeved on the column component 11F, and two ends of the driving component 119 can respectively abut against the pivoting assembly 116 and the column component 11F (as shown FIG. 8). However, the present invention is not limited to this embodiment. For example, in another embodiment, two ends of the driving component can be respectively inserted into the column component and the pivoting body.

Specifically, please referred to FIG. 8, the column component 11F and the driving component 119 are located at the first side of the lever 117. An end of the driving component 119 is inserted into the column component 11F, and another end of the driving component 119 abuts against a surface of the stopping structure 1163 of the pivoting assembly 116. The column component 11F can be moved along a disengaging direction T1 perpendicular to the unlocking direction L1 and the insertion direction D1 for resiliently compressing the driving component 119 along the disengaging direction T1, or the column component 11F can be pivoted along a second pivoting direction P2 opposite to the first pivoting direction P1 for pivoting the pivoting assembly 116 along the second pivoting direction P2 and resiliently twisting the driving component 119. The base 111 includes an accommodating portion 1111 for at least partially receiving the column component 11F. A length of the column component 11F along a direction parallel to the disengaging direction T1 can be less than a length of the accommodating portion 1111 along the direction parallel to the disengaging direction T1, so that the column component 11F is movable relative to the accommodating portion 1111 along the disengaging direction T1. The column component 11F includes a first locking structure 11F1. The accommodating portion 1111 includes a second locking structure 11111. A pivoting movement of the column component 11F is restrained by an engagement of the first locking structure 11F1 and the second locking structure 11111, and a disengagement of the first locking structure 11F1 and the second locking structure 11111 allows the pivoting movement of the column component 11F when the column component 11F moves along the disengaging direction T1 relative to the accommodating portion 1111 to disengage the first locking structure 11F1 from the second locking structure 11111.

In this embodiment, the length of the accommodating portion 1111 along the direction parallel to the disengaging direction T1 can be less than a length of the driving component 119 along the direction parallel to the disengaging direction T1. In such a way, the driving component 119 can not only be resiliently twisted by the column component 11F to generate a first resilient force for driving the pivoting assembly 116 to pivot along the first pivoting direction P1 but also be resiliently compressed along the disengaging direction T1 to generate a second resilient force for driving the column component 11F to move along an engaging direction T2 opposite to the disengaging direction T1 relative to the accommodating portion 1111 due to the first locking structure 11F1 engaged with the second locking structure 11111.

Besides, in order for easy manual operation, an operating recess 11F2 is formed on the column component 11F for insertion of a hand tool, such as a screw driver or a hex key, to move the column component 11F along the disengaging direction T1 for disengaging the first locking structure 11F1 from the second locking structure 11111 and to pivot the column component 11F along the second pivoting direction P2 for pivoting the pivoting assembly 116 along the second pivoting direction P2 and resiliently twisting the driving component 119.

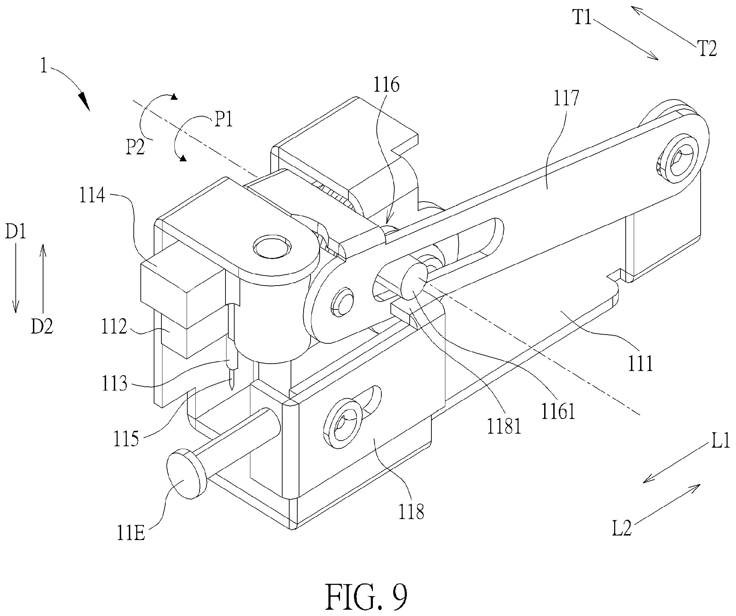

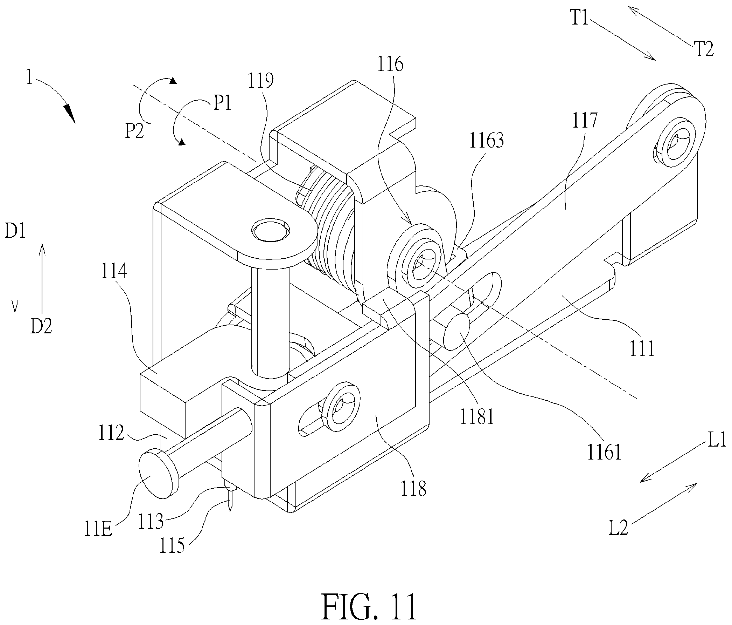

Please refer to FIG. 9 to FIG. 12. FIG. 9 is a partial diagram of the medical device 1 in a first state according to the embodiment of the present invention. FIG. 10 is a partial diagram of the medical device 1 in a second state according to the embodiment of the present invention. FIG. 11 is a partial diagram of the medical device 1 in a third state according to the embodiment of the present invention. FIG. 12 is a partial diagram of the medical device 1 in a fourth state according to the embodiment of the present invention. As shown in FIG. 8 and FIG. 9, in order to set the medical device 1 in the first state, the column component 11F can be operated by the hand tool to move along the disengaging direction T1 for resiliently compressing the driving component 119, so as to disengage the first locking structure 11F1 from the second locking structure 11111. When the first locking structure 11F1 is disengaged from the second locking structure 11111, the column component 11F can be further operated to pivot along the second pivoting direction P2 for driving the driving component 119 to move to a first position as shown in FIG. 9 and for resiliently twisting the driving component 119. Afterwards, the column component 11F can be driven by the driving component 119 to move along the engaging direction T2, and the operating component 11E can be operated to slide the triggering component 118 to a locking position as shown in FIG. 9, so that the abutting structure 1181 of the triggering component 118 engages with the protruding structure 1161 of the pivoting assembly 116.

When it is desired to use the medical device 1, the operating component 11E can be operated to drive the triggering component 118 to slide from the locking position as shown in FIG. 9 to an unlocking position as shown in FIG. 10 for slidably disengaging the abutting structure 1181 of the triggering component 118 from the protruding structure 1161 of the pivoting assembly 116. When the abutting structure 1181 of the triggering component 118 is disengaged from the protruding structure 1161 of the pivoting assembly 116, the driving component 119 can drive the pivoting assembly 116 to pivot along the first pivoting direction P1 until the stopping structure 1163 contacts with the base 111, i.e., to pivot from the first position as shown in FIG. 10 through a second position as shown in FIG. 11 to a third position as shown in FIG. 12. When the pivoting assembly 116 pivots from the first position as shown in FIG. 10 to the second position as shown in FIG. 11, the pivoting assembly 116 drives the lever 117 to drive the needle holder 114 to slide from the original position along the insertion direction D1 to push the cannula holder 112 to slide relative the base 111 together with the needle holder 114 by the pushing structure 1141, which is shown in FIG. 4, for the insertion of the needle 115 and the cannula 113 into the patient's body along the insertion direction D1. When the pivoting assembly 116 pivots from the second position as shown in FIG. 11 to the third position as shown in FIG. 11, the pivoting assembly 116 drives the lever 117 to drive the needle holder 114 to slide back to the original position along the retraction direction D2, so as to drive the needle 115 to retract relative to the cannula 113 along the retraction direction D2 for withdrawing the needle 115 from the patient's body along the retraction direction D2 and leaving the cannula 113 inserted into the patient's body.

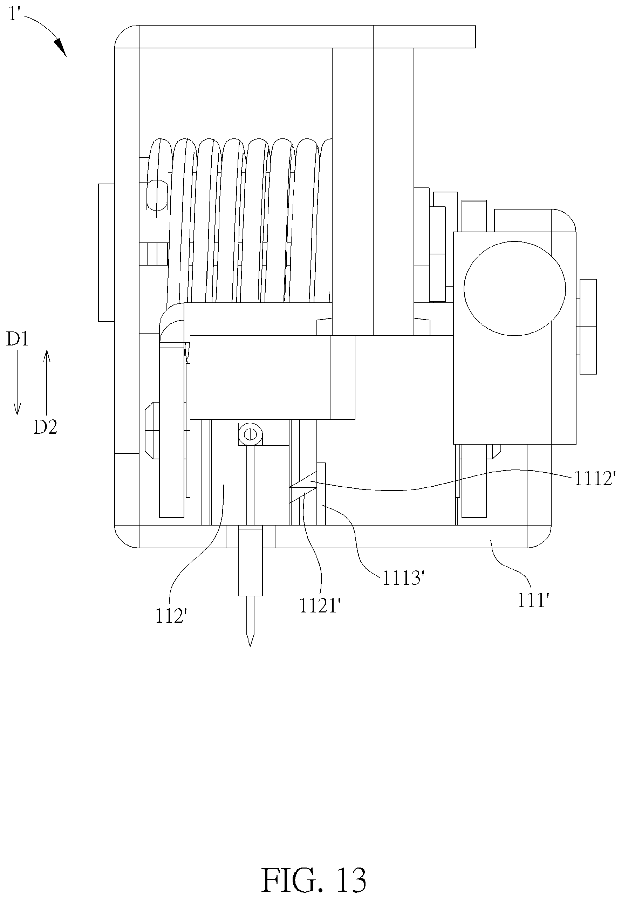

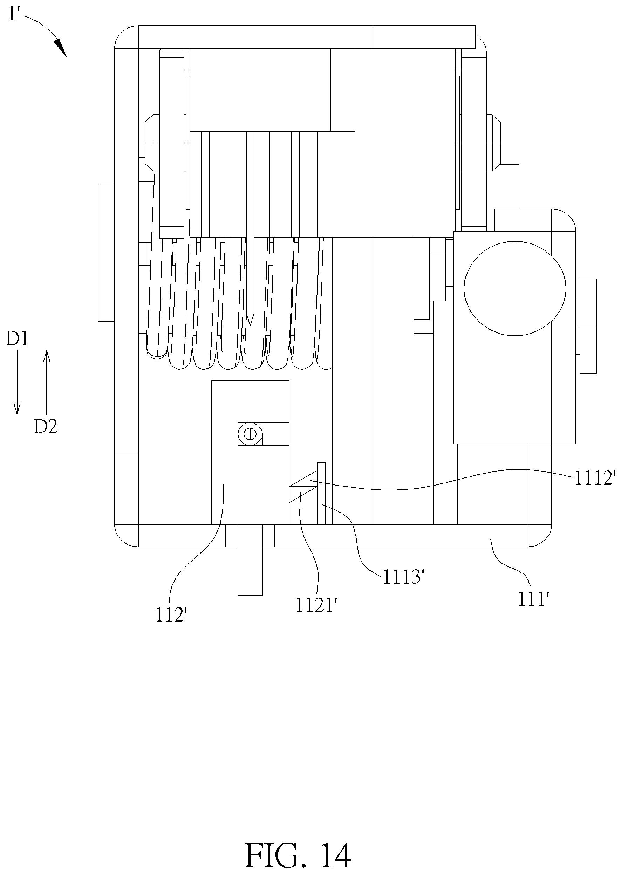

Please refer to FIG. 13 and FIG. 14. FIG. 13 and FIG. 14 are partial diagrams of a medical device 1' in different states according to another embodiment of the present invention. As shown in FIG. 13 and FIG. 14, different from the above-mentioned embodiment, the cannula holder 112' includes a first engaging structure 1121'. The base 111' includes a second engaging structure 1112' and a resilient arm 1113'. The second engaging structure 1112' is disposed on the resilient arm 1113'. The resilient arm 1113' can be resiliently deformed by an abutment of the first engaging structure 1121' and the second engaging structure 1112' for allowing the first engaging structure 1121' to pass over the second engaging structure 1112' when the cannula holder 112' moves along the insertion direction D1, and a sliding movement of the cannula holder 112' along the retraction direction D2 is restrained by an engagement of the first engaging structure 1121' and the second engaging structure 1112'. Other details of this embodiment are similar to the ones of the above-mentioned embodiment. Detailed description is omitted herein for simplicity.

In contrast to the prior art, the present invention has less parts and compact size. Besides, in the present invention, when it is desired to use the medical device for drug injection or fluid drainage, it only has to operate the operating component to drive the triggering component to slide for slidably disengaging the abutting structure of the triggering component from the protruding structure of the pivoting assembly, so that the cannula can be inserted into the patient's body. Therefore, the present invention has easy operation and precise insertion and retraction function.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. A medical insertion mechanism (11) comprising:
a base (111, 111');
a needle holder (114) disposed on the base (111, 111') and slidable relative to the base (111, 111') along an insertion direction (D1) or a retraction direction (D2) opposite to the insertion direction (D1), the needle holder (114) comprising a pushing structure (1141);
a needle (115) disposed on the needle holder (114);
a cannula holder (112, 112') arranged at a position corresponding to the needle holder (114), the pushing structure (1141) being configured to abut against the cannula holder (112, 112') along the insertion direction (D1);
a cannula (113) disposed on the cannula holder (112, 112');
a pivoting assembly (116) disposed on the base (111, 111') and pivotable relative to the base (111, 111') from a first position through a second position to a third position;
a lever (117) movably disposed on the base (111, 111') and movably engaged with the pivoting assembly (116) and the needle holder (114);
a triggering component (118) disposed on the base (111, 111'), the triggering component (118) comprising an abutting structure (1181) configured to slidably engage with or disengage from the pivoting assembly (116); and
a driving component (119) configured to drive the pivoting assembly (116) to pivot from the first position through the second position to the third position when the abutting structure (1181) slidably disengages from the pivoting assembly (116);
wherein when the pivoting assembly (116) pivots from the first position to the second position, the lever (117) drives the needle holder (114) and the cannula holder (112, 112') to slide along the insertion direction (D1), so as to drive the needle (115) and the cannula (113) to move along the insertion direction (D1) together;
wherein when the pivoting assembly (116) pivots from the second position to the third position, the lever (117) drives the needle holder (114) to slide along the retraction direction (D2) to drive the needle (115) to retract relative to the cannula (113);
further **characterized by**:
a column component (11F) detachably installed on the base (111, 111') and for resiliently deforming the driving component (119), the driving component (119) being a spring at least partially sleeved on the column component (11F), and two ends of the driving component (119) respectively abutting against the pivoting assembly (116) and the column component (11F);
wherein the column component (11F), the driving component (119), the cannula holder (112, 112') and the needle holder (114) are located at a first side of the lever (117), and the abutting structure (1181) is located at a second side of the lever (117).

2. The medical insertion mechanism (11) of claim 1, **characterized in that** the lever (117) is located between the pivoting assembly (116) and the triggering component (118).

3. The medical insertion mechanism (11) of any of claims 1 and 2, **characterized in that** the pivoting assembly (116) is engaged with a longitudinal slot (1174) formed on a middle portion (1173) of the lever (117) between a first end (1171) and a second end (1172) of the lever (117), the longitudinal slot (1174) is adjacent to the second end (1172) of the lever (117) and away from the first end (1171) of the lever (117), and the pivoting assembly (116) comprises a protruding structure (1161) passing through the longitudinal slot (1174) and movable relative to the longitudinal slot (1174).

4. The medical insertion mechanism (11) of any of claims 1 and 2, **characterized in that** one and another one of an elongated hole (1175) and a circular hole (1176) are respectively formed on a first end (1171) and a second end (1172) of the lever (117), the medical insertion mechanism (11) further comprises a connecting pin (11A) disposed on the base (111, 111') and a driving pin (11B) disposed on the needle holder (114), the connecting pin (11A) passes through the one of the elongated hole (1175) and the circular hole (1176) and is movable relative to the one of the elongated hole (1175) and the circular hole (1176), and the driving pin (11B) passes through the another one of the elongated hole (1175) and the circular hole (1176) and is movable relative to the another one of the elongated hole (1175) and the circular hole (1176).

5. The medical insertion mechanism (11) of any of claims 1, 2 and 4, **characterized in that** the pivoting assembly (116) comprises a protruding structure (1161) and a stopping structure (1163), the abutting structure (1181) is configured to abut against the protruding structure (1161), and the stopping structure (1163) is configured to contact with the base (111, 111') for stopping the pivoting assembly (116) when the pivoting assembly (116) pivots from the first position to the third position.

6. The medical insertion mechanism (11) of claim 5, **characterized in that** a longitudinal slot (1174) is formed on a middle portion (1173) of the lever (117) between a first end (1171) and a second end (1172) of the lever (117), the pivoting assembly (116) further comprises a pivoting body (1162), the abutting structure (1181) and the pivoting body (1162) are located at two opposite sides of the lever (117), and the protruding structure (1161) protrudes from the pivoting body (1162), passes through the longitudinal slot (1174) and is movable relative to the longitudinal slot (1174).

7. The medical insertion mechanism (11) of any of claims 1 to 6, further **characterized by** a guiding component (11C) disposed on the base (111, 111') and configured to guide the needle holder (114) and the cannula holder (112, 112') to slide.

8. The medical insertion mechanism (11) of claim 7, **characterized in that** the lever (117) is engaged with a portion of the needle holder (114) located between the pivoting assembly (116) and the guiding component (11C).

9. The medical insertion mechanism (11) of any of claims 1 to 8, **characterized in that** the cannula holder (112, 112') comprises a first engaging structure (1121'), the base (111, 111') comprises a second engaging structure (1112'), and a sliding movement of the cannula holder (112, 112') is restrained by an engagement of the first engaging structure (1121') and the second engaging structure (1112').

10. The medical insertion mechanism (11) of any one of claims 1 to 9, **characterized in that** the base (111, 111') comprises an accommodating portion (1111) for at least partially receiving the column component (11F), a length of the column component (11F) is less than a length of the accommodating portion (1111), the column component (11F) comprises a first locking structure (11F1), the accommodating portion (1111) comprises a second locking structure (11111), a pivoting movement of the column component (11F) is restrained by an engagement of the first locking structure (11F1) and the second locking structure (11111), a disengagement of the first locking structure (11F1) and the second locking structure (11111) allows the pivoting movement of the column component (11F) when the column component (11F) moves relative to the accommodating portion (1111) to disengage the first locking structure (11F1) from the second locking structure (11111), the length of the accommodating portion (1111) is less than a length of the driving component (119), and the driving component (119) drives the column component (11F) to move relative to the accommodating portion (1111) to engage the first locking structure (11F1) with the second locking structure (11111).

11. The medical insertion mechanism (11) of any of claims 1 to 10, **characterized in that** the lever (117) is driven by the pivoting assembly (116) to drive the needle holder (114) to slide from an original position along the insertion direction (D1) and then back to the original position along the retraction direction (D2) when the pivoting assembly (116) pivots from the first position to the third position.

12. The medical insertion mechanism (11) of any of claims 1 to 11, **characterized in that** a sliding slot (1182) is formed on the triggering component (118), the medical insertion mechanism (11) further comprises a sliding pin (11D) disposed on the base (111, 111') and an operating component (11E) connected to the triggering component (118), and the sliding pin (11D) passes through the sliding slot (1182) and is movable relative to the sliding slot (1182).

13. A medical device (1, 1') comprising:
a case (12); and
**characterized in that** the medical device (1, 1') further comprises the medical insertion mechanism (11) of any of claims 1-12;
wherein the base (111, 111') is disposed on the case (12).

## Patentansprüche

1. Medizinischer Einführmechanismus (11), umfassend:
eine Basis (111, 111');
einen Nadelhalter (114), der auf der Basis (111, 111') angeordnet ist und relativ zur Basis (111, 111') entlang einer Einführrichtung (D1) oder einer der Einführrichtung (D1) entgegengesetzten Rückzugsrichtung (D2) verschiebbar ist, worin der Nadelhalter (114) einen Schubaufbau (1141) umfasst;
eine Nadel (115), die auf dem Nadelhalter (114) angeordnet ist;
einen Kanülenhalter (112, 112'), der an einer Position angeordnet ist, die dem Nadelhalter (114) entspricht, worin der Schubaufbau (1141) ausgestaltet ist, entlang der Einführrichtung (D1) an dem Kanülenhalter (112, 112') anzuliegen;
eine Kanüle (113), die auf dem Kanülenhalter (112, 112') angeordnet ist;
einer Schwenkanordnung (116), die auf der Basis (111, 111') angeordnet ist und relativ zur Basis (111, 111') aus einer ersten Position über eine zweite Position in eine dritte Position schwenkbar ist;
einen Hebel (117), der beweglich auf der Basis (111, 111') angeordnet ist und beweglich mit der Schwenkanordnung (116) und dem Nadelhalter (114) in Eingriff steht;
eine Auslösekomponente (118), die auf der Basis (111, 111') angeordnet ist, worin die Auslösekomponente (118) eine Anschlagstruktur (1181) umfasst, die ausgestaltet ist, gleitend mit der Schwenkanordnung (116) in Eingriff gebracht oder von dieser gelöst zu werden; und
eine Antriebskomponente (119), die ausgestaltet ist, die Schwenkanordnung (116) anzutreiben, um von der ersten Position über die zweite Position in die dritte Position zu schwenken, wenn sich die Anschlagstruktur (1181) gleitend von der Schwenkanordnung (116) löst;
wobei, wenn sich die Schwenkanordnung (116) von der ersten Position in die zweite Position schwenkt, der Hebel (117) den Nadelhalter (114) und den Kanülenhalter (112, 112') dazu, antreibt, entlang der Einführrichtung (D1) zu gleiten, um die Nadel (115) und die Kanüle (113) dazu anzutreiben, sich gemeinsam entlang der Einführrichtung (D1) zu bewegen;
wobei, wenn die Schwenkanordnung (116) von der zweiten Position in die dritte Position schwenkt, der Hebel (117) den Nadelhalter (114) antreibt, um entlang der Rückzugsrichtung (D2) zu gleiten, um die Nadel (115) anzutreiben, sich relativ zur Kanüle (113) zurückzuziehen;
weiter **gekennzeichnet durch**:
eine Säulenkomponente (11F), die abnehmbar an der Basis (111, 111') angebracht ist und zum elastischen Verformen der Antriebskomponente (119) dient, worin die Antriebskomponente (119) eine Feder ist, die zumindest teilweise auf die Säulenkomponente (11F) aufgeschoben ist, und zwei Enden der Antriebskomponente (119) jeweils an der Schwenkanordnung (116) und der Säulenkomponente (11F) anliegen;
worin sich die Säulenkomponente (11F), die Antriebskomponente (119), der Kanülenhalter (112, 112') und der Nadelhalter (114) auf einer ersten Seite des Hebels (117) befinden und sich die Anschlagstruktur (1181) auf einer zweiten Seite des Hebels (117) befindet.

2. Medizinischer Einführmechanismus (11) nach Anspruch 1, **dadurch gekennzeichnet, dass** sich der Hebel (117) zwischen der Schwenkanordnung (116) und der Auslösekomponente (118) befindet.

3. Medizinischer Einführmechanismus (11) nach einem der Ansprüche 1 und 2, **dadurch gekennzeichnet, dass** die Schwenkanordnung (116) mit einem Längsschlitz (1174) in Eingriff steht, der an einem mittleren Abschnitt (1173) des Hebels (117) zwischen einem ersten Ende (1171) und einem zweiten Ende (1172) des Hebels (117) ausgebildet ist, der Längsschlitz (1174) benachbart zum zweiten Ende (1172) des Hebels (117) und entfernt vom ersten Ende (1171) des Hebels (117) angeordnet ist, und die Schwenkanordnung (116) eine vorstehende Struktur (1161) umfasst, die durch den Längsschlitz (1174) hindurchgeht und relativ zum Längsschlitz (1174) bewegbar ist.

4. Medizinischer Einführmechanismus (11) nach einem der Ansprüche 1 und 2, **dadurch gekennzeichnet, dass** eines von einem Langloch (1175) und einem kreisförmigen Loch (1176) entsprechend an einem ersten Ende (1171) und einem zweiten Ende (1172) des Hebels (117) ausgebildet sind, worin der medizinische Einführmechanismus (11) ferner einen Verbindungsstift (11A), der an der Basis (111, 111') angeordnet ist, und einen Antriebsstift (11B) umfasst, der an der Nadelhalterung (114) angeordnet ist, worin der Verbindungsstift (11A) durch das eine von dem Langloch (1175) und dem kreisförmigen Loch (1176) hindurchgeht und relativ zu dem einen von Langloch (1175) und dem kreisförmigen Loch (1176) bewegbar ist, und der Antriebsstift (11B) durch das andere von dem Langloch (1175) und dem kreisförmigen Loch (1176) hindurch geht und relativ zu dem anderen von dem Langlöcher (1175) und dem kreisförmigen Loch (1176) bewegbar ist.

5. Medizinischer Einführmechanismus (11) nach einem der Ansprüche 1, 2 und 4, **dadurch gekennzeichnet, dass** die Schwenkanordnung (116) eine vorstehende Struktur (1161) und eine Stoppstruktur (1163) umfasst, worin die Anschlagstruktur (1181) ausgestaltet ist, an der vorstehenden Struktur (1161) anzuliegen, und die Stoppstruktur (1163) ausgestaltet ist, mit der Basis (111, 111') in Kontakt zu kommen, um die Schwenkanordnung (116) anzuhalten, wenn die Schwenkanordnung (116) von der ersten Position in die dritte Position schwenkt.

6. Medizinischer Einführmechanismus (11) nach Anspruch 5, **dadurch gekennzeichnet, dass** ein Längsschlitz (1174) an einem mittleren Abschnitt (1173) des Hebels (117) zwischen einem ersten Ende (1171) und einem zweiten Ende (1172) des Hebels (117) ausgebildet ist, worin die Schwenkanordnung (116) ferner einen Schwenkkörper (1162) umfasst, die Anschlagstruktur (1181) und der Schwenkkörper (1162) sich an zwei abgewandten Seiten des Hebels (117) befinden und die vorstehende Struktur (1161) aus dem Schwenkkörper (1162) herausragt, durch den Längsschlitz (1174) verläuft und relativ zum Längsschlitz (1174) bewegbar ist.

7. Medizinischer Einführmechanismus (11) nach einem der Ansprüche 1 bis 6, ferner **gekennzeichnet durch** eine Führungskomponente (11C), die an der Basis (111, 111') angeordnet und ausgestaltet ist, den Nadelhalter (114) und den Kanülenhalter (112, 112') zum Gleiten zu führen.

8. Medizinischer Einführmechanismus (11) nach Anspruch 7, **dadurch gekennzeichnet, dass** der Hebel (117) mit einem Abschnitt des Nadelhalters (114) in Eingriff steht, der sich zwischen der Schwenkanordnung (116) und der Führungskomponente (11C) befindet.

9. Medizinischer Einführmechanismus (11) nach einem der Ansprüche 1 bis 8, **dadurch gekennzeichnet, dass** der Kanülenhalter (112, 112') eine erste Eingriffsstruktur (1121') umfasst, die Basis (111, 111') eine zweite Eingriffsstruktur (1112') umfasst und eine Gleitbewegung des Kanülenhalters (112, 112') durch einen Eingriff der ersten Eingriffsstruktur (1121') und der zweiten Eingriffsstruktur (1112') eingeschränkt wird.

10. Medizinischer Einführmechanismus (11) nach einem der Ansprüche 1 bis 9, **dadurch gekennzeichnet, dass** die Basis (111, 111') einen Aufnahmeabschnitt (1111) zum zumindest teilweisen Aufnehmen der Säulenkomponente (11F) umfasst, worin eine Länge der Säulenkomponente (11F) geringer ist als eine Länge des Aufnahmeabschnitts (1111), die Säulenkomponente (11F) eine erste Verriegelungsstruktur (11F1) umfasst, der Aufnahmeabschnitt (1111) eine zweite Verriegelungsstruktur (11111) umfasst, eine Schwenkbewegung der Säulenkomponente (11F) durch ein Eingriff der ersten Verriegelungsstruktur (11F1) und der zweiten Verriegelungsstruktur (11111) eingeschränkt wird, ein Ausrücken der ersten Verriegelungsstruktur (11F1) und der zweiten Verriegelungsstruktur (11111) die Schwenkbewegung der Säulenkomponente (11F) ermöglicht, wenn sich die Säulenkomponente (11F) relativ zum Aufnahmeabschnitt (1111) bewegt, um die erste Verriegelungsstruktur (11F1) aus der zweiten Verriegelungsstruktur (11111) auszurücken, die Länge des Aufnahmeabschnitts (1111) geringer ist als die Länge der Antriebskomponente (119), und die Antriebskomponente (119) die Säulenkomponente (11F) antreibt, um sich relativ zum Aufnahmeabschnitt (1111) zu bewegen, um die erste Verriegelungsstruktur (11F1) mit der zweiten Verriegelungsstruktur (11111) in Eingriff zu bringen.

11. Medizinischer Einführmechanismus (11) nach einem der Ansprüche 1 bis 10, **dadurch gekennzeichnet, dass** der Hebel (117) durch die Schwenkanordnung (116) angetrieben wird, um den Nadelhalter (114) zu bewegen, sodass er aus einer Ausgangsposition entlang der Einführrichtung (D1) gleitet und dann entlang der Rückzugsrichtung (D2) in die Ausgangsposition zurückgleitet, wenn die Schwenkanordnung (116) von der ersten Position in die dritte Position schwenkt.

12. Medizinischer Einführmechanismus (11) nach einem der Ansprüche 1 bis 11, **dadurch gekennzeichnet, dass** ein Gleitschlitz (1182) an der Auslösekomponente (118) ausgebildet ist, worin der medizinische Einführmechanismus (11) ferner einen Gleitstift (11D) umfasst, der an der Basis (111, 111') angeordnet ist, und eine Betätigungskomponente (11E) umfasst, die mit der Auslösekomponente (118) verbunden ist, und der Gleitstift (11D) durch den Gleitschlitz (1182) hindurchgeht und relativ zum Gleitschlitz (1182) beweglich ist.

13. Medizinisches Gerät (1, 1') mit:
einem Gehäuse (12); und
**dadurch gekennzeichnet, dass** das medizinische Gerät (1, 1') ferner den medizinischen Einführmechanismus (11) nach einem der Ansprüche 1 bis 12 umfasst;
worin die Basis (111, 111') an dem Gehäuse (12) angeordnet ist.

## Revendications

1. Mécanisme d'insertion médical (11) comprenant:
une base (111, 111');
un porte-aiguille (114) disposé sur la base (111, 111') et pouvant coulisser par rapport à la base (111, 111') le long d'une direction d'insertion (D1) ou d'une direction de retrait (D2) opposée à la direction d'insertion (D1), le porte-aiguille (114) comprenant une structure de poussée (1141);
une aiguille (115) disposée sur le porte-aiguille (114);
un porte-canule (112, 112') disposé à une position correspondant au porte-aiguille (114), la structure de poussée (1141) étant configurée pour venir en butée contre le porte-canule (112, 112') le long de la direction d'insertion (D1);
une canule (113) disposée sur le porte-canule (112, 112');
un ensemble pivotant (116) disposé sur la base (111, 111') et pouvant pivoter par rapport à la base (111, 111') d'une première position à une troisième position en passant par une deuxième position;
un levier (117) disposé de manière mobile sur la base (111, 111') et engagé de manière mobile avec l'ensemble pivotant (116) et le porte-aiguille (114);
un composant de déclenchement (118) disposé sur la base (111, 111'), le composant de déclenchement (118) comprenant une structure de butée (1181) configurée pour s'engager de manière coulissante avec l'ensemble pivotant (116) ou s'en désengager; et
un composant d'entraînement (119) configuré pour entraîner l'ensemble pivotant (116) à pivoter de la première position à la troisième position en passant par la deuxième position lorsque la structure de butée (1181) se désengage de manière coulissante de l'ensemble pivotant (116);
dans lequel, lorsque l'ensemble pivotant (116) pivote de la première position à la deuxième position, le levier (117) entraîne le porte-aiguille (114) et le porte-canule (112, 112') à coulisser le long de la direction d'insertion (D1), de manière à entraîner l'aiguille (115) et la canule (113) à se déplacer ensemble le long de la direction d'insertion (D1);
dans lequel, lorsque l'ensemble pivotant (116) pivote de la deuxième position à la troisième position, le levier (117) entraîne le porte-aiguille (114) à coulisser le long de la direction de rétraction (D2) pour entraîner l'aiguille (115) à se rétracter par rapport à la canule (113);
**caractérisé en outre par**:
un composant de colonne (11F) installé de manière amovible sur la base (111, 111') et destiné à déformer de manière élastique le composant d'entraînement (119), le composant d'entraînement (119) étant un ressort au moins partiellement enfilé sur le composant de colonne (11F), et les deux extrémités du composant d'entraînement (119) venant respectivement en butée contre l'ensemble pivotant (116) et le composant de colonne (11F);
dans lequel le composant de colonne (11F), le composant d'entraînement (119), le porte-canule (112, 112') et le porte-aiguille (114) sont situés sur un premier côté du levier (117), et la structure de butée (1181) est située sur un deuxième côté du levier (117).

2. Mécanisme d'insertion médicale (11) selon la revendication 1, **caractérisé en ce que** le levier (117) est situé entre l'ensemble pivotant (116) et le composant de déclenchement (118).

3. Mécanisme d'insertion médical (11) de l'une quelconque des revendications 1 et 2, **caractérisé en ce que** l'ensemble pivotant (116) est engagé dans une fente longitudinale (1174) formée sur une partie centrale (1173) du levier (117) entre une première extrémité (1171) et une deuxième extrémité (1172) du levier (117), la fente longitudinale (1174) est adjacente à la deuxième extrémité (1172) du levier (117) et éloignée de la première extrémité (1171) du levier (117), et l'ensemble pivotant (116) comprend une structure en saillie (1161) passant à travers la fente longitudinale (1174) et mobile par rapport à la fente longitudinale (1174).

4. Mécanisme d'insertion médicale (11) de l'une quelconque des revendications 1 et 2, **caractérisé en ce qu'**un trou allongé (1175) et un trou circulaire (1176) sont respectivement formés sur une première extrémité (1171) et une deuxième extrémité (1172) du levier (117), le mécanisme d'insertion médicale (11) comprenant en outre une broche de connexion (11A) disposée sur la base (111, 111') et une broche d'entraînement (11B) disposée sur le porte-aiguille (114), la broche de connexion (11A) passant à travers l'un des trous allongés (1175) et des trous circulaires (1176) et est mobile par rapport à l'un des trous allongés (1175) et des trous circulaires (1176), et la broche d'entraînement (11B) passe à travers l'autre des trous allongés (1175) et des trous circulaires (1176) et est mobile par rapport à l'autre des trous allongés (1175) et des trous circulaires (1176).

5. Mécanisme d'insertion médical (11) de l'une quelconque des revendications 1, 2 et 4, **caractérisé en ce que** l'ensemble pivotant (116) comprend une structure en saillie (1161) et une structure d'arrêt (1163), la structure de butée (1181) est configurée pour venir en butée contre la structure en saillie (1161), et la structure d'arrêt (1163) est configurée pour entrer en contact avec la base (111, 111') afin d'arrêter l'ensemble pivotant (116) lorsque l'ensemble pivotant (116) pivote de la première position à la troisième position.

6. Mécanisme d'insertion médicale (11) selon la revendication 5, **caractérisé en ce qu'**une fente longitudinale (1174) est formée sur une partie centrale (1173) du levier (117) entre une première extrémité (1171) et une deuxième extrémité (1172) du levier (117), l'ensemble pivotant (116) comprenant en outre un corps pivotant (1162), la structure de butée (1181) et le corps pivotant (1162) sont situés sur deux côtés opposés du levier (117), et la structure en saillie (1161) fait saillie à partir du corps pivotant (1162), passe à travers la fente longitudinale (1174) et est mobile par rapport à la fente longitudinale (1174).

7. Mécanisme d'insertion médical (11) de l'une quelconque des revendications 1 à 6, **caractérisé en outre par** un composant de guidage (11C) disposé sur la base (111, 111') et configuré pour guider le porte-aiguille (114) et le porte-canule (112, 112') afin qu'ils coulissent.

8. Mécanisme d'insertion médical (11) de la revendication 7, **caractérisé en ce que** le levier (117) est engagé avec une partie du porte-aiguille (114) située entre l'ensemble pivotant (116) et le composant de guidage (11C).

9. Mécanisme d'insertion médical (11) selon l'une quelconque des revendications 1 à 8, **caractérisé en ce que** le porte-canule (112, 112') comprend une première structure d'engagement (1121'), la base (111, 111') comprend une deuxième structure d'engagement (1112'), et un mouvement de coulissement du porte-canule (112, 112') est limité par un engagement de la première structure d'engagement (1121') et de la deuxième structure d'engagement (1112').

10. Mécanisme d'insertion médicale (11) selon l'une quelconque des revendications 1 à 9, **caractérisé en ce que** la base (111, 111') comprend une partie d'accueil (1111) destinée à recevoir au moins partiellement le composant de colonne (11F), une longueur du composant de colonne (11F) est inférieure à une longueur de la partie d'accueil (1111), le composant de colonne (11F) comprend une première structure de verrouillage (11F1), la partie d'accueil (1111) comprend une deuxième structure de verrouillage (11111), un mouvement de pivotement du composant de colonne (11F) est limité par un engagement de la première structure de verrouillage (11F1) et de la deuxième structure de verrouillage (11111), un désengagement de la première structure de verrouillage (11F1) et de la deuxième structure de verrouillage (11111) permet le mouvement de pivotement du composant de colonne (11F) lorsque le composant de colonne (11F) se déplace par rapport à la partie d'accueil (1111) pour désengager la première structure de verrouillage (11F1) de la deuxième structure de verrouillage (11111), la longueur de la partie d'accueil (1111) est inférieure à la longueur du composant d'entraînement (119), et le composant d'entraînement (119) entraîne le composant de colonne (11F) à se déplacer par rapport à la partie d'accueil (1111) pour engager la première structure de verrouillage (11F1) avec la deuxième structure de verrouillage (11111).

11. Mécanisme d'insertion médicale (11) de l'une quelconque des revendications 1 à 10, **caractérisé en ce que** le levier (117) est entraîné par l'ensemble pivotant (116) pour entraîner le porte-aiguille (114) à coulisser depuis une position d'origine le long de la direction d'insertion (D1), puis à revenir à la position d'origine le long de la direction de rétraction (D2) lorsque l'ensemble pivotant (116) pivote de la première position à la troisième position.

12. Mécanisme d'insertion médical (11) selon l'une quelconque des revendications 1 à 11, **caractérisé en ce qu'**une fente coulissante (1182) est formée sur le composant de déclenchement (118), le mécanisme d'insertion médical (11) comprenant en outre une broche coulissante (11D) disposée sur la base (111, 111') et un composant de commande (11E) relié au composant de déclenchement (118), et la broche coulissante (11D) passe à travers la fente coulissante (1182) et est mobile par rapport à la fente coulissante (1182).

13. Dispositif médical (1, 1') comprenant:
un boîtier (12); et
**caractérisé en ce que** le dispositif médical (1, l') comprend en outre le mécanisme d'insertion médical (11) de l'une quelconque des revendications 1 à 12;
dans lequel la base (111, 111') est disposée sur le boîtier (12).
